# DEMANDE DE BREVET EUROPEEN

(11) **EP 4 756 840 A1**
(43) Date de publication de la demande: **10.06.2026**
(21) Numéro de dépôt: 25220621.4
(22) Date de dépôt: 04.12.2025
(51) Int. Cl.: G21G 1/00

(54) **PROCÉDÉ DE PRODUCTION DE PLOMB-212 DE QUALITÉ MÉDICALE**

(30) Priorité: 05.12.2024 FR 2413470
(71) Demandeur: Orano Med Manufacturing, 92320 Chatillon (FR)
(72) Inventeur: DUREAU, Rémy, 87510 SAINT GENCE (FR); FLEURAT, Mélissa, 87510 SAINT JOUVENT (FR); MALTERRE, Alexandre, 87000 LIMOGES (FR); TORGUE, Julien, GAITHERSBURG, MD 20878 (US)
(74) Mandataire: Santarelli

(57) **Abrégé**

L'invention concerne un procédé permettant de produire du plomb-212 de qualité médicale, comprenant les étapes de :
a) production de plomb-212 par désintégration radioactive de radium-224 dans au moins une première colonne de chromatographie contenant une première phase stationnaire sur laquelle est fixé le radium-224 ;
b) élution du plomb-212 ainsi produit de la première phase stationnaire ;
c) chargement de l'éluat ainsi obtenu dans une deuxième colonne de chromatographie contenant une deuxième phase stationnaire pour fixer le plomb-212 sur la deuxième phase stationnaire ;
d) lavage(s) de la deuxième phase stationnaire ; puis
e) élution du plomb-212 de la deuxième phase stationnaire ;
et caractérisé en ce que :
- l'étape b) comprend une circulation dans ladite au moins une première colonne de chromatographie d'une solution aqueuse A1 comprenant de 0,8 mol/L à 1,6 mol/L d'un chlorure, éventuellement avec au plus 200 mmol/L d'HCl ; et
- l'étape d) comprend une circulation dans la deuxième colonne de chromatographie d'une solution aqueuse A2 comprenant de 0,01 mol/L à 1 mol/L du chlorure.

Applications : fabrication de radiopharmaceutiques à base de plomb-212, utiles en médecine nucléaire.

## Description

### Domaine technique

L'invention se rapporte au domaine de la production d'isotopes radioactifs, aussi dits radioisotopes.

Plus spécifiquement, elle se rapporte à un procédé qui permet de produire du plomb-212 présentant un très haut degré de pureté radiologique, ce qui le rend pleinement adapté à un usage médical.

Ce procédé est donc susceptible de trouver des applications dans la fabrication de radiopharmaceutiques à base de plomb-212, utiles en médecine nucléaire et, en particulier, en radiothérapie alpha ciblée pour le traitement des cancers.

### État de la technique antérieure

Le plomb-212 est un isotope radioactif rare du plomb, qui fait l'objet depuis plusieurs années de recherches prometteuses, notamment pour le traitement par radiothérapie alpha ciblée, aussi appelée alphathérapie ciblée, de cancers et, en particulier, des cancers du pancréas, des ovaires, du côlon, du sein et de la prostate.

Le plomb-212 fait également partie des radioisotopes dont il a été montré qu'ils présentent un intérêt en imagerie médicale, notamment pour réaliser des examens par tomographie par émission monophotonique couplée à un scanner.

Dans les deux cas, l'utilisation du plomb-212 implique que celui-ci soit injecté au patient sous la forme d'un radiopharmaceutique, c'est-à-dire d'un produit dans lequel il est lié, typiquement par l'intermédiaire d'un agent chélatant, à une molécule capable de cibler très spécifiquement les cellules que l'on souhaite détruire (s'il s'agit d'alphathérapie ciblée) ou observer (s'il s'agit d'imagerie médicale), telle qu'un peptide.

Pour ce faire, le plomb-212 doit satisfaire à des exigences de qualité extrêmement strictes et, notamment, de pureté radiologique, celle-ci devant être idéalement au moins égale à 99,95 %.

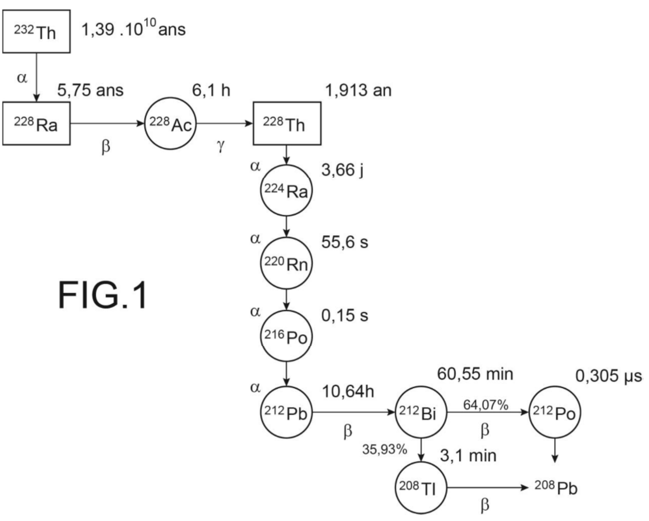

À cet égard, on précise que l'on entend par pureté radiologique d'un radioisotope tel que le plomb-212, la pureté que ce radioisotope présente vis-à-vis des radioisotopes dont il est issu par désintégration radioactive (c'est-à-dire ses ascendants) ainsi que vis-à-vis des autres radioisotopes qui ne font pas partie de sa chaîne de désintégration radioactive, et non pas la pureté que ce radioisotope présente vis-à-vis des radioisotopes auxquels il donne naissance par sa propre désintégration radioactive (c'est-à-dire ses descendants). Comme l'illustre la figure 1 jointe en annexe, qui représente la chaîne de désintégration, aussi dite de décroissance, radioactive du thorium-232, le plomb-212 appartient à la famille radioactive du thorium-232 dont il est un produit de filiation. Il est également un produit de filiation du thorium-228 et du radium-224 qui s'inscrivent, dans cette chaîne, entre le thorium-232 et le plomb-212.

Pour produire du plomb-212 de qualité médicale, c'est-à-dire répondant aux exigences précitées de pureté radiologique, il a été proposé dans les demandes internationales PCT WO-A-2013/174949 et WO-A-2017/093069, ci-après références **[1]** et **[2],** des procédés qui comprennent schématiquement :
- la production de plomb-212 par désintégration radioactive de radium-224 dans un générateur qui comprend une phase stationnaire sur laquelle est fixé le radium-224 et qui sera appelé plus simplement générateur Ra-224/Pb-212 dans ce qui suit ;
- l'élution du plomb-212 ainsi produit de la phase stationnaire du générateur Ra-224/Pb-212, puis
- la purification du plomb-212 ainsi élué par une chromatographie en phase liquide. Dans ces références, sont également décrits des appareils spécialement conçus pour une mise en œuvre automatisée, en système fermé, de ces procédés.

Il se trouve que les références **[1]** et **[2]** prévoient toutes deux de réaliser l'élution du plomb-212 de la phase stationnaire du générateur Ra-224/Pb-212 avec une solution aqueuse comprenant de 1,5 mol/L à 2,5 mol/L d'un acide fort du type acide chlorhydrique ou acide nitrique, l'acide utilisé dans les exemples de ces références étant l'acide chlorhydrique à hauteur de 2 mol/L. Cette élution conduit à l'obtention d'un éluat fortement acide qui est ensuite chargé dans la colonne de chromatographie servant à la purification du plomb-212.

Or, l'utilisation d'acide chlorhydrique aux concentrations préconisées dans les références **[1]** et **[2]** et, en particulier, d'acide chlorhydrique 2M pose des problèmes de corrosion des équipements susceptibles d'être utilisés pour la production du plomb-212, comme les appareils d'élution automatisés dont certains composants (corps de vannes, visseries ou roulements par exemple) sont sensibles aux acides ainsi que des surfaces avoisinantes en aciers telles que celles des boites à gants ou des chaines blindées avec, à la clé, un taux de maintenance élevé.

Par ailleurs, dans la référence **[2],** il est envisagé que le procédé puisse comprendre, en amont de la production du plomb-212 dans le générateur Ra-224/Pb-212, une étape visant à produire le radium-224 lui-même par désintégration radioactive de thorium-228 dans un générateur qui comprend une phase stationnaire sur laquelle est fixé ce thorium et qui sera appelé plus simplement générateur Th-228/Ra-224 dans ce qui suit. Il est précisé que, dans ce cas, l'élution du radium-224 de cette phase stationnaire en vue de sa récupération est réalisée avec une solution aqueuse acide telle qu'une solution d'acide chlorhydrique. La concentration en acide chlorhydrique de cette solution d'élution n'est pas mentionnée mais on peut déduire de la référence **[2]** que cette concentration est comprise entre 1 mol/L et 3 mol/L puisque l'éluat comprenant le radium-224, qui est ensuite utilisé pour fixer ce radium sur la phase stationnaire du générateur Ra-224/Pb-212, présente une concentration en acide chlorhydrique comprise entre 1 mol/L et 3 mol/L et préférentiellement de 2 mol/L. L'utilisation d'une telle solution d'élution pose donc les mêmes problèmes de corrosion que ceux évoqués précédemment. En outre, la courbe de rétention du thorium-228 par la résine DGA (Triskem International) dont l'utilisation est préconisée dans la référence **[2],** en tant que phase stationnaire du générateur Th-228/Ra-224, montre une pente très importante en milieu chlorhydrique 1M à 3M. Il en résulte que la moindre approximation de la molarité de cet acide expose à un risque élevé de fuites du thorium-228 et/ou de diminution de la durée de vie du générateur.

Compte-tenu de ce qui précède, les inventeurs se sont fixés pour objectif de fournir un procédé de production de plomb-212 qui, tout en présentant les mêmes performances que les procédés décrits dans les références **[1]** et **[2],** notamment en termes de rendement et de qualité du plomb-212 produit, soit exempt des inconvénients évoqués ci-avant.

Ils se sont également fixé pour objectif d'améliorer les performances d'élution du plomb-212 lors de sa purification par chromatographie en phase liquide et, plus spécifiquement, de parvenir à réduire le volume de solution d'élution nécessaire pour éluer la totalité de l'activité disponible du plomb-212.

Ils se sont en outre fixé pour objectif que ce procédé puisse être mis en œuvre de façon automatisée, par exemple au moyen de l'un des appareils décrits dans les références **[1]** et **[2].**

### Exposé de l'invention

Ces objectifs sont remplis par l'invention qui propose un procédé de production de plomb-212 de qualité médicale, comprenant au moins les étapes de :
a) production de plomb-212 dans au moins un générateur Ra-224/Pb-212, c'est-à-dire par désintégration radioactive du radium-224 présent dans au moins une première colonne de chromatographie contenant une première phase stationnaire sur laquelle est fixé le radium-224 ;
b) élution du plomb-212 ainsi produit de la première phase stationnaire pour obtenir un éluat comprenant du plomb-212 non purifié ;
c) chargement de l'éluat ainsi obtenu dans une deuxième colonne de chromatographie contenant une deuxième phase stationnaire pour fixer le plomb-212 présent dans l'éluat sur la deuxième phase stationnaire ;
d) lavage(s) de la deuxième phase stationnaire pour éliminer les impuretés radioactives susceptibles d'avoir été retenues par la deuxième phase stationnaire sans éliminer le plomb-212 ; et
e) élution du plomb-212 de la deuxième phase stationnaire, moyennant quoi le plomb-212 de qualité médicale est obtenu en solution aqueuse ;
et qui est caractérisé en ce que :
- l'étape b) comprend une circulation dans ladite au moins une première colonne de chromatographie d'une solution aqueuse A1 comprenant de 0,8 mol/L à 1,6 mol/L d'un chlorure, seul ou en mélange avec au plus 200 mmol/L d'acide chlorhydrique ; et
- l'étape d) comprend une circulation dans la deuxième colonne de chromatographie d'une solution aqueuse A2 comprenant de 0,01 mol/L à 1 mol/L du chlorure.

Ainsi, selon l'invention, la solution aqueuse, qui est utilisée à l'étape b) pour éluer le plomb-212 de la phase stationnaire du ou des générateurs Ra-224/Pb-212, est une solution qui comprend un chlorure et dans laquelle de l'acide chlorhydrique peut être présent mais à une concentration infiniment moindre que celle préconisée dans les références **[1]** et **[2].** Par voie de conséquence, il en est de même pour l'éluat qui est chargé dans la deuxième colonne de chromatographie à l'étape c).

De plus, le(s) lavage(s) de la phase stationnaire sur laquelle est fixé le plomb-212 - ou étape d) - est (sont) réalisé(s) avec une solution aqueuse comprenant un chlorure et non, avec une solution aqueuse d'acide chlorhydrique comme décrit dans les exemples des références **[1]** et **[2].**

Les risques de corrosion des équipements et surfaces avoisinantes sont ainsi supprimés ou, à tout le moins, drastiquement réduits avec, pour conséquence, un allègement du taux de maintenance et, par là même, des coûts de production du plomb-212 et ceci sans que le niveau de pureté radiologique obtenu en soit affecté.

Conformément à l'invention, les première et deuxième phases stationnaires sont préférentiellement du même type que celles qui sont utilisées dans les références **[1]** et **[2]** pour respectivement produire du plomb-212 dans le générateur Ra-224/Pb-212 et purifier ensuite ce plomb, c'est-à-dire que :
- la première phase stationnaire est, de préférence, une résine échangeuse de cations qui retient le radium, quel qu'en soit l'isotope, mais ne retient pas le plomb en présence d'ions chlorures, quel qu'en soit l'isotope, telle qu'une résine constituée de particules d'un polymère organique, tel qu'un poly(styrène-*co*-divinylbenzène), sur lequel sont greffés des groupes sulfoniques, SO₃H, du type de celle qui est disponible auprès de la société Bio-Rad sous le nom commercial AG^{™} MP-50, tandis que
- la deuxième phase stationnaire est, de préférence, une résine d'extraction qui est constituée de particules d'un support inerte imprégné d'un éther-couronne, tel qu'un dicyclohexano-18-couronne-6 ou un dibenzo-18-couronne-6 dont les groupes cyclohexyles ou benzyles sont substitués par un ou plusieurs groupes alkyles en C₁ à C₁₂, à chaîne droite ou ramifiée ; ainsi, la deuxième phase stationnaire peut notamment être une résine dont le support inerte est imprégné de 4,4'(5')-di-*tert*-butylcyclohexano-18-couronne-6 en solution dans l'isodécanol, telle que celle disponible auprès de la société Triskem International sous le nom commercial Résine PB.

Le chlorure présent dans les solutions aqueuses A1 et A2 peut être choisi parmi de nombreux sels comprenant au moins un anion chlorure. En particulier, il peut s'agir d'un chlorure d'un métal et, notamment, d'un chlorure d'un métal alcalin, tel qu'un chlorure de sodium ou de potassium, ou d'un métal alcalino-terreux, tel qu'un chlorure de calcium ou de magnésium, ou encore de chlorure d'ammonium. Parmi ces sels, préférence est donnée au chlorure de magnésium.

Conformément à l'invention, la solution aqueuse A1 comprend, de préférence, de 0,8 mol/L à 1,2 mol/L et, mieux encore, 1,0 mol/L de chlorure de magnésium, seul ou en mélange avec au plus 50 mmol/L d'acide chlorhydrique, cet acide étant, s'il est présent, préférentiellement à une concentration de 1 mmol/L.

La solution aqueuse A2 comprend, elle, préférentiellement de 0,1 mol/L à 1 mol/L et, mieux encore, 1 mol/L de chlorure de magnésium.

Sachant que, comme toute colonne de chromatographie, la deuxième colonne présente deux extrémités opposées, respectivement appelées tête et queue de colonne, l'étape d) comprend la circulation d'un premier volume de solution aqueuse A2 de la tête de colonne vers la queue de colonne, puis la circulation d'un second volume de solution aqueuse A2, identique ou différent du premier volume, de la queue de colonne vers la tête de colonne.

L'étape e) comprend, elle, une circulation dans la deuxième colonne de chromatographie d'une solution aqueuse A3, laquelle présente avantageusement un pH compris entre 5 et 9 et comprend, de préférence, un ou plusieurs agents complexants ou chélatants - les deux termes étant considérés ici comme synonymes - et/ou agents antioxydants.

Le ou les agents complexants ou chélatants peuvent être notamment choisis parmi :
- l'acétate d'ammonium que l'on utilise, de préférence, à une concentration allant de 0,15 mol/L à 1 mol/L ;
- l'acide citrique et ses sels tels que les citrates d'un métal alcalin (comme le citrate monosodique, le citrate disodique ou le citrate trisodique), les citrates d'un métal alcalino-terreux (comme le citrate monocalcique, le citrate dicalcique ou le citrate tricalcique) ou encore les citrates d'ammonium comme le citrate d'ammonium monobasique, le citrate d'ammonium dibasique ou le citrate d'ammonium tribasique, que l'on utilise préférentiellement à une concentration allant de 10 mmol/L à 200 mmol/L, préférence étant donnée à l'acide citrique ; et
- les chélateurs qui sont typiquement utilisés dans la préparation de produits destinés à la médecine nucléaire et, notamment les dérivés du cyclène, c'est-à-dire du 1,4,7,10-tétraazacyclododécane, tels que le DOTA (ou acide 1,4,7,10-tétraazacyclodécane-1,4,7,10-tétraacétique) ou le DOTAM (ou amide 1,4,7,10-tétraazacyclodécane-1,4,7,10-tétraacétique), ce qui permet alors de gagner un temps précieux dans la fabrication de radiopharmaceutiques à base de plomb-212 compte-tenu que la demi-vie de ce dernier n'est que de 10,6 heures ; si de tels chélateurs sont utilisés, alors ils le sont préférentiellement à hauteur de 0,2 µmol/L à 200 µmol/L.

Le ou agents antioxydants peuvent, quant à eux, être notamment choisis parmi l'acide ascorbique et l'acide citrique, que l'on utilise, de préférence, à une concentration allant de 10 mmol/ à 200 mmol/L.

Avantageusement, la solution aqueuse A3 comprend de l'acétate d'ammonium et de l'acide citrique (qui fait à la fois office d'agent complexant et d'agent antioxydant) et/ou du DOTAM. Ainsi, par exemple, la solution aqueuse A3 peut comprendre 0,4 mol/L d'acétate d'ammonium et 75 mmol/L d'acide citrique ou bien 0,4 mol/L d'acétate d'ammonium, 75 mmol/L d'acide citrique et 2 µmol/L de DOTAM.

Quoi qu'il en soit, la solution aqueuse A3 est, de préférence, mise à circuler dans la deuxième colonne de chromatographie de la queue de colonne vers la tête de colonne. Avantageusement, le procédé est mis en œuvre en utilisant *m* générateurs Ra-224/Pb-212, c'est-à-dire *m* premières colonnes de chromatographie contenant chacune une première phase stationnaire sur laquelle est fixé le radium-224, *m* étant un nombre entier au moins égal à 2, typiquement compris entre 2 et 4, et une seule deuxième colonne de chromatographie.

Dans ce cas, les *m* premières colonnes de chromatographie peuvent être disposées en parallèle, auquel cas on réalise :
- les étapes a) et b) dans chacune des *m* premières colonnes de chromatographie, moyennant quoi on obtient *m* éluats comprenant du plomb-212 non purifié que l'on collecte séparément ou ensemble pour former un mélange des *m* éluats ;
- l'étape c) en chargeant les *m* éluats ou le mélange des *m* éluats ainsi obtenus dans la deuxième colonne de chromatographie contenant la deuxième phase stationnaire ;
- l'étape d) de lavage de la deuxième phase stationnaire ; et
- l'étape e) d'élution du plomb-212 de la deuxième phase stationnaire.

Ainsi, par le chargement de ces *m* éluats (séparément ou sous forme d'un mélange) dans la deuxième colonne de chromatographie, il est possible d'augmenter la quantité de plomb-212 non purifié qui se fixe sur la deuxième phase stationnaire et, de ce fait, de concentrer le plomb-212 dans la solution aqueuse issue de l'élution prévue à l'étape e). En variante, les *m* premières colonnes de chromatographie peuvent être connectées en série, auquel cas on réalise :
- les étapes a) et b) dans chacune des *m* premières colonnes de chromatographie, l'étape b) étant réalisée en faisant circuler la solution aqueuse A1 successivement dans lesdites *m* premières colonnes de chromatographie, moyennant quoi on obtient, au sortir de la *m*^{ième} de ces colonnes, un éluat comprenant du plomb-212 non purifié ;
- l'étape c) en chargeant l'éluat ainsi obtenu dans la deuxième colonne de chromatographie contenant la deuxième phase stationnaire ;
- l'étape d) de lavage de la deuxième phase stationnaire ; et
- l'étape e) d'élution du plomb-212 de la deuxième phase stationnaire.

Ainsi, avec cette variante, il s'est avéré possible d'éluer le plomb-212 des *m* générateurs Ra-224/Pb-212 en utilisant un volume de solution aqueuse A1 notablement plus faible que celui qui serait nécessaire pour éluer la même quantité de plomb-212 de *m* générateurs Ra-224/Pb-212 ayant la même charge en plomb-212 mais qui ne seraient pas connectés en série avec, à la clé, une économie de réactifs, une concentration du plomb-212 non purifié dans l'unique éluat obtenu au sortir du *m*^{ième} générateur Ra-224/Pb-212 et, par là même, une meilleure fixation de ce plomb sur la phase stationnaire de la deuxième colonne de chromatographie (puisque présent dans un volume plus faible) et une concentration du plomb-212 dans la solution aqueuse issue de l'élution prévue à l'étape e).

Quelle que soit la variante, on utilise préférentiellement deux ou trois premières colonnes de chromatographie, c'est-à-dire que *m* = 2 ou 3.

Conformément à l'invention, le procédé comprend préférentiellement de plus, avant l'étape a), les étapes de :
i) production de radium-224 dans au moins un générateur Th-228/Ra-224, c'est-à-dire par désintégration radioactive du thorium-228 dans au moins une troisième colonne de chromatographie comprenant une troisième phase stationnaire sur laquelle est fixé le thorium-228 ; et
ii) élution du radium-224 ainsi produit de la troisième phase stationnaire pour obtenir un éluat comprenant du radium-224, l'élution comprenant une circulation dans ladite au moins une troisième colonne de chromatographie d'une solution aqueuse A0 comprenant de 0,4 mol/L à 1 mol/L d'acide nitrique ; puis
iii) chargement de l'éluat ainsi obtenu dans ladite au moins une première colonne de chromatographie pour fixer le radium-224 présent dans l'éluat sur la première phase stationnaire.

Conformément à l'invention, la troisième phase stationnaire est, de préférence, une résine d'extraction qui constituée de particules d'un polymère organique, tel qu'un polyméthacrylate ou un poly(styrène-*co*-divinylbenzène), imprégné d'un ligand des actinides tel qu'un diglycolamide tétraalkylé (par exemple, le *N,N,N',N'*-tétraoctyl-diglycolamide ou TODGA), un acide dialkylphosphorique (par exemple, l'acide di(2-éthylhexyl)phosphorique ou HDEHP) ou un oxyde de trialkylphosphine (par exemple, l'oxyde de trioctylphosphine ou TOPO).

Ainsi, la troisième phase stationnaire peut notamment être une résine dont le polymère est imprégné de TODGA, telle que celle disponible auprès de la société Triskem International sous le nom commercial Résine DGA Normal.

De préférence, la solution aqueuse A0 comprend de 0,5 mol/L à 0,75 mol/L et, mieux encore, 0,5 mol/L d'acide nitrique.

Avantageusement, on utilise *n* générateurs Th-228/Ra-224, c'est-à-dire *n* troisièmes colonnes de chromatographie contenant chacune une troisième phase stationnaire sur laquelle est fixé le thorium-228, *n* étant un nombre entier au moins égal à 2, typiquement compris entre 2 et 5.

Dans ce cas, les *n* troisièmes colonnes de chromatographie peuvent être disposées en parallèle, auquel cas on réalise :
- les étapes i) et ii) dans chacune des *n* troisièmes colonnes de chromatographie, moyennant quoi on obtient *n* éluats comprenant du radium-224 que l'on collecte séparément ou ensemble pour former un mélange des *n* éluats ; et
- l'étape iii) en chargeant les *n* éluats ou le mélange des *n* éluats ainsi obtenus dans ladite au moins une première colonne de chromatographie.

Ceci permet d'augmenter la quantité de radium-224 qui se fixe à l'étape iii) sur la phase stationnaire contenue dans ladite au moins une première colonne de chromatographie et, de ce fait, augmenter la quantité de plomb-212 produit par désintégration radioactive du radium-224 à l'étape a).

En variante, les *n* troisièmes colonnes de chromatographie peuvent être connectées en série, auquel cas on réalise :
- les étapes i) et ii) dans chacune des *n* troisièmes colonnes de chromatographie, l'étape ii) étant réalisée en faisant circuler la solution aqueuse A0 successivement dans lesdites *n* troisièmes colonnes de chromatographie, moyennant quoi on obtient, au sortir de la *n*^{ième} de ces colonnes, un éluat comprenant du radium-224 ; et
- l'étape iii) en chargeant l'éluat ainsi obtenu dans ladite au moins une première colonne de chromatographie.

Là également, cette variante permet d'éluer le radium-224 des *n* générateurs Th-228/Ra-224 en utilisant un volume de solution aqueuse A0 notablement plus faible que celui qui serait nécessaire pour éluer la même quantité de radium-224 de *n* générateurs Th-228/Ra-224 ayant la même charge en radium-224 mais qui ne seraient pas connectés en série avec, à la clé, une économie de réactifs, une concentration du radium-224 dans l'unique éluat obtenu au sortir du *n*^{ième} générateur Th-228/Ra-224 et, par voie de conséquence, une meilleure fixation de ce radium sur la phase stationnaire de ladite au moins première colonne de chromatographie ou, autrement dit, dudit au moins un générateur Ra-224/Pb-212.

Quelle que soit la variante, on utilise préférentiellement deux ou trois troisièmes colonnes de chromatographie, c'est-à-dire que *n* = 2 ou 3.

D'autres caractéristiques et avantages du procédé de l'invention apparaitront à la lecture du complément de description qui suit et qui se rapporte à des modes de mise en œuvre de ce procédé.

Il va de soi que ces modes de mise en œuvre ne sont donnés qu'à titre illustratif et ne constituent en aucun cas une limitation de l'objet de l'invention.

### Brève description des figures

[Fig. 1], déjà commentée, représente la chaîne de désintégration radioactive du thorium-232.
[Fig. 2] représente schématiquement un premier mode de mise en œuvre du procédé de l'invention.
[Fig.3] représente schématiquement un deuxième mode de mise en œuvre du procédé de l'invention.

### Exposé détaillé de modes de mise en œuvre particuliers

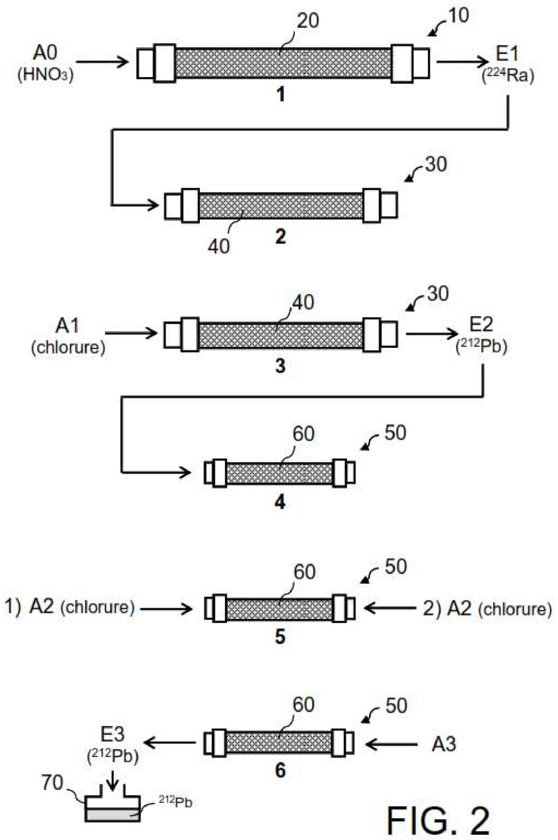

On se réfère à la figure 2 qui illustre schématiquement les différentes étapes, notées 1 à 6, d'un premier mode de mise en œuvre du procédé de l'invention, dans lequel sont utilisés un seul générateur Th-228/Ra-224 et un seul générateur Ra-224/Pb-212.

Dans ce mode de mise en œuvre, le point de départ du procédé est donc représenté par un générateur Th-228/Ra-224, noté 10 sur la figure 2. Ce générateur comprend une colonne de chromatographie dont la phase stationnaire, notée 20, est constituée de particules de résine DGA Normal (Triskem International) et sur laquelle est fixé du thorium-228.

Le thorium-228 présent dans le générateur 10 ayant été laissé décroître pour produire du radium-224, le procédé comprend les étapes suivantes :
1. l'élution du radium-224 ainsi produit de la phase stationnaire 20 au moyen d'une solution aqueuse A0 d'acide nitrique pour obtenir un éluat E1 comprenant du radium-224 ;
2. la préparation d'un générateur Ra-224/Pb-212, noté 30, par chargement de l'éluat E1 dans une colonne de chromatographie, dont la phase stationnaire 40 est constituée de particules de résine AG^{™} MP-50 (Bio-Rad), pour fixer le radium-224 présent dans cet éluat sur la phase stationnaire 40 ;
3. le radium-224 présent dans le générateur 30 ayant été laissé décroître pour produire du plomb-212, l'élution de ce plomb de la phase stationnaire 40 au moyen d'une solution aqueuse A1 d'un chlorure, seul ou en mélange avec de l'acide chlorhydrique très faiblement concentré, pour obtenir un éluat E2 comprenant du plomb-212 ;
4. le chargement de l'éluat E2 dans une colonne de chromatographie, notée 50, dont la phase stationnaire 60 est constituée de particules de Résine PB (Triskem International), pour fixer le plomb-212 présent dans cet éluat sur cette phase stationnaire ;
5. deux lavages successifs de la phase stationnaire 60 pour retirer de la colonne 50, et notamment du volume interstitiel de cette phase stationnaire, les traces de radioisotopes autres que le plomb-212 susceptibles d'avoir été retenus dans cette colonne à l'étape précédente, chacun de ces lavages étant effectué avec une solution aqueuse A2 d'un chlorure mais en sens inverse l'un de l'autre ;
6. l'élution du plomb-212 de la phase stationnaire 60 au moyen d'une solution aqueuse A3 présentant un pH allant de 5 à 9 et comprenant un ou plusieurs agents complexants et/ou agents antioxydants pour obtenir un éluat E3 comprenant du plomb 212 purifié et le recueil de cet éluat dans un réceptacle, noté 70, qui peut-être du type bécher, fiole, ou analogue, comme illustré sur la figure 2, mais qui peut également être une seringue connectée à la queue de la colonne 50.

Toutes ces étapes, qui sont détaillées ci-après, sont effectuées à température ambiante, c'est-à-dire à une température de 20 °C à 25 °C.

Par ailleurs, toutes les solutions utilisées sont, de préférence, de grade Optima^{™} ou préparées à partir de réactifs de grade Optima^{™} ou de grade Métaux Traces (ou *Trace Metals grade* en langue anglaise).

Sauf indication contraire, les solutions aqueuses qui sont mises à circuler dans les colonnes 10, 30 et 50 le sont de la tête de colonne vers la queue de colonne.

### * Étape 1 :

Comme précédemment indiqué, cette étape consiste à éluer, de la phase stationnaire 20 du générateur 10, le radium-224 ayant été produit par désintégration radioactive du thorium-228 fixé sur cette phase stationnaire.

Ce générateur comprend une colonne de chromatographie qui présente, par exemple, un volume de lit, ou BV pour *Bed Volume,* allant de 5 mL à 100 mL et qui est remplie de particules de résine DGA Normal, par exemple à hauteur de 2 g à 40 g de particules selon le BV de la colonne.

Ce type de résine retient le thorium, quel qu'en soit l'isotope, mais ne retient pas le radium, quel qu'en soit l'isotope.

L'élution du radium-224 est réalisée en faisant circuler dans le générateur 10 plusieurs BV de solution aqueuse A0 - laquelle comprend de 0,5 mol/L à 0,75 mol/L et, de préférence, 0,5 mol/L d'acide nitrique - et ce, à un débit qui est, par exemple, de 0,25 BV/min.

Est ainsi obtenu l'éluat E1.

### * Étape 2 :

Le générateur 30 est préparé en utilisant une colonne de chromatographie de dimensions plus réduites que celles de la colonne de chromatographie du générateur 10 et qui présente, par exemple, un BV allant de 0,5 mL à 10 mL, et qui est remplie de particules de résine AG^{™} MP-50, par exemple à hauteur de 300 mg à 5 g de particules selon le BV de cette colonne, et faisant circuler l'éluat E1 dans cette colonne.

En milieu chlorures, la résine AG^{™} MP-50 retient le radium, quel qu'en soit l'isotope, mais ne retient pas le plomb, quel qu'en soit l'isotope.

Le chargement de l'éluat E1 dans le générateur 30 est réalisé à un débit qui est, par exemple, de 4 BV/min.

### * Étape 3 :

Comme précédemment indiqué, cette étape consiste, après une période de temps pendant laquelle on a laissé le générateur 30 produire du plomb-212 par désintégration radioactive du radium-224 présent dans ce générateur, à éluer le plomb-212 ainsi produit de la phase stationnaire 40.

Pour ce faire, plusieurs BV de solution aqueuse A1 sont mis à circuler dans le générateur 30, par exemple à un débit de 4 BV/min, cette solution aqueuse comprenant de 0,8 mol/L à 1,6 mol/L d'un chlorure, avantageusement de chlorure de magnésium, et éventuellement de l'acide chlorhydrique, cet acide, s'il est présent, présente une concentration au plus de 200 mmol/L, de préférence au plus de 50 mmol/L et, mieux encore, de 1 mmol/L.

Est ainsi obtenu l'éluat E2.

### * Étape 4 :

Le plomb-212 présent dans l'éluat E2 ne répondant pas encore au critère de pureté radiologique requis pour un usage médical, les étapes 4, 5 et 6 visent à purifier le plomb-212 vis-à-vis de ses ascendants, et notamment vis-à-vis du radium-224, au moyen d'une colonne de chromatographie remplie de résine PB.

La colonne de chromatographie 50 que l'on utilise pour ce faire, de dimensions plus réduites que la colonne de chromatographie du générateur 30, présente, par exemple, un BV allant de 0,1 mL à 1 mL et est remplie de 35 mg à 450 mg de particules de Résine PB. Le chargement de l'éluat E2 dans la colonne 50 est réalisé en faisant circuler cet éluat dans la colonne 50 à un débit qui est, par exemple, de 4 BV/min.

### * Étape 5 :

Les deux lavages successifs prévus à cette étape sont réalisés avec la même solution aqueuse A2 mais en sens inverse l'un de l'autre.

Cette solution aqueuse comprend de 25 mmol/L à 75 mmol/L du même chlorure que celui présent dans la solution aqueuse A1 utilisée à l'étape 3 ci-avant, par exemple 50 mmol/L de chlorure de magnésium.

Le premier lavage est réalisé en faisant circuler dans la colonne 50, de la tête de colonne vers la queue de colonne, plusieurs BV de solution aqueuse A2, par exemple à un débit de 6 BV/min, tandis que le deuxième lavage est réalisé en faisant circuler dans cette colonne, de préférence, le même nombre de BV de solution aqueuse A2 et au même débit mais de la queue de colonne vers la tête de colonne.

### * Étape 6 :

L'élution du plomb-212 de la phase stationnaire 60 est réalisée en faisant circuler dans la colonne 50, de la queue de colonne vers la tête de colonne, plusieurs BV de solution aqueuse A3, laquelle présente un pH compris entre 5 et 9 et comprend un ou plusieurs agents complexants et/ou agents antioxydants.

La solution aqueuse A3 est, par exemple, une solution aqueuse qui présente un pH de 6,5 et comprend 0,4 mol/L d'acétate d'ammonium et 75 mmol/L d'acide citrique.

Elle est mise à circuler dans la colonne 50 à un débit, par exemple, de 6 BV/min.

L'éluat E3 ainsi obtenu en tête de colonne est recueilli dans le récipient 70 et le plomb-212 présent dans cet éluat présente une pureté radiologique vis-à-vis de ses ascendants permettant son utilisation à des fins médicales.

Comme précédemment indiqué, le procédé tel qu'il vient d'être décrit peut également être mis en œuvre en utilisant *n* générateurs Th-228/Ra-224, *n* étant par exemple égal à 3, disposés en parallèle ou connectés en série, ainsi qu'avec *m* générateurs Ra-224/Pb-212, *m* étant par exemple égal à 2, également disposés en parallèle ou connectés en série.

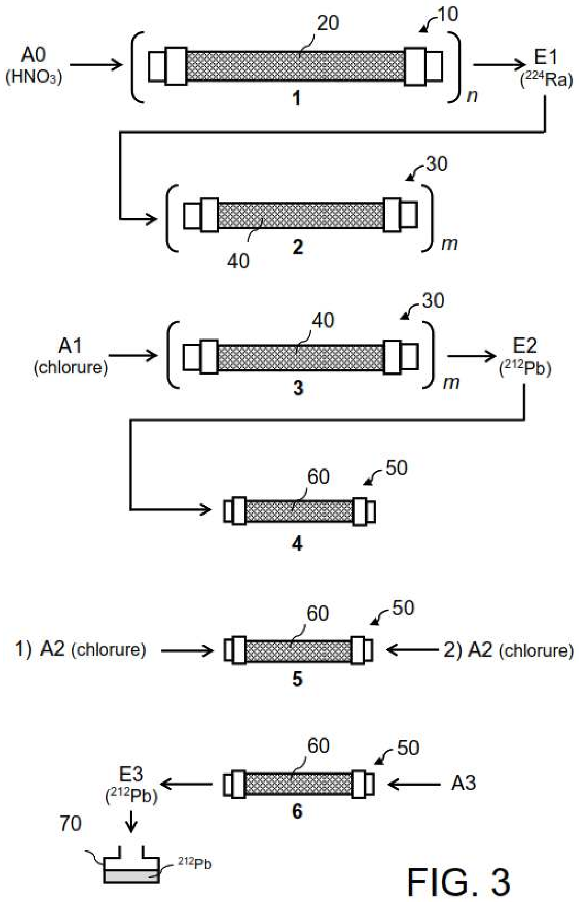

Ainsi, par exemple, comme illustré sur la figure 3 qui illustre un mode de mise en œuvre du procédé dans lequel sont utilisés *n* générateurs Th-228/Ra-224 connectés en série et *m* générateurs Ra-224/Pb-212 connectés en série, ce mode de mise en œuvre ne diffère de celui représenté sur la figure 2 qu'en ce que :
- le thorium-228 ayant été laissé décroître dans les *n* générateurs 10 pour produire du radium-224, l'étape 1 est réalisée en faisant circuler la solution aqueuse A0 d'acide nitrique successivement dans ces *n* générateurs 10 ou, autrement dit, en introduisant cette solution dans le premier des *n* générateurs 10 et en la faisant circuler dans les *n-1* générateurs 10 suivants, ce qui conduit à l'obtention d'un unique éluat E1 comprenant du radium-224 au sortir du *n*^{ième} générateur 10 ;
- l'étape 2 est réalisée en faisant circuler l'éluat E1 ainsi obtenu successivement dans les *m* générateurs 30 ou, autrement dit, en introduisant cet éluat dans le premier des *m* générateurs 30 et en la faisant circuler dans les *m-1* générateurs 30 suivants ; et
- le radium-224 ayant été laissé décroître dans les *m* générateurs 30 pour produire du plomb-212, l'étape 3 est réalisée en faisant circuler la solution aqueuse A1 successivement dans ces *m* générateurs 30 ou, autrement dit, en introduisant cette solution dans le premier des *m* générateurs 30 et en la faisant circuler dans les *m-1* générateurs 30 suivants, ce qui conduit à l'obtention d'un unique éluat E2 comprenant du plomb-212 non purifié au sortir du *m*^{ième} générateur 30.

### Exemple 1 - MgCl₂ 1 M / CLG BV 1,25 mL / CLL BV 0,173 mL :

Les étapes 1 à 6 du premier mode de mise en œuvre décrit ci-avant ont été mises en œuvre en utilisant :
** **Étape 1*** : 30 mL d'une solution aqueuse A0 comprenant 0,5 mol/L d'acide nitrique, à un débit de 5 mL/min, pour éluer le radium-224 de la phase stationnaire d'un générateur 10 de BV égal à 9,8 mL et contenant 3,7 g de résine DGA ;
** **Étape 2 :*** une colonne 30 de BV égal à 1,25 mL, contenant 0,65 g de résine AGTM MP-50 et que l'on a chargée, à un débit de 5 mL/min, avec 25,4 mL de l'éluat E1 obtenu à l'étape précédente, cet éluat comprenant 167 MBq de radium-224 ;
** **Étape 3 :*** après 20 heures pendant lesquelles on a laissé le radium-224 produire du plomb-212, 10 mL d'une solution aqueuse A1 comprenant 1 mol/L de MgCl2, à un débit de 0,5 mL/min ;
** **Étape 4 :*** une colonne 50 de BV égal à 0,173 mL (préalablement lavée avec 1 mL d'une solution aqueuse à 1,0 mol/L de MgCl2, à un débit de 0,5 mL/min), contenant 73 ± 5 mg de résine PB et que l'on a chargée avec l'éluat E2 obtenu à l'étape 3, à un débit de 0,5 mL/min ;
** **Étape 5 :*** 2 fois 1,5 mL d'une solution aqueuse A2 comprenant 1,0 mol/L de MgCl₂, à un débit de 0,5 mL/min ;
** **Étape 6 :*** 1,6 mL d'une solution aqueuse A3 comprenant 0,4 mol/L d'acétate d'ammonium et 75 mmol/L d'acide citrique, à un débit de 0,5 mL/min.

Une solution aqueuse contenant 96,8 MBq de plomb-212 a ainsi été obtenue.

Ce plomb-212 a présenté, vis-à-vis du radium-224, une pureté radiologique de plus de 99,999 %, le radium-224 n'ayant pas été détecté après une semaine.

### Exemple 2 - MgCl₂ 1 M / CLG BV = 5,0 mL / CLL BV = 0,63 mL :

Les étapes 1 à 6 du premier mode de mise en œuvre décrit ci-avant ont été mises en œuvre en utilisant :
** **Étape 1*** : 30 mL d'une solution aqueuse A0 comprenant 0,5 mol/L d'acide nitrique, à un débit de 5 mL/min, pour éluer le radium-224 de la phase stationnaire d'un générateur 10 de BV égal à 9,8 mL et contenant 3,7 g de résine DGA ;
** **Étape 2 :*** une colonne 30 de BV égal à 5 mL, contenant 2,43 g de résine AGTM MP-50 et que l'on a chargée, à un débit de 5 mL/min, avec 37,8 mL de l'éluat E1 obtenu à l'étape précédente, cet éluat comprenant 116 MBq de radium-224 ;
** **Étape 3 :*** après 23 heures pendant lesquelles on a laissé le radium-224 produire du plomb-212, 30 mL d'une solution aqueuse A1 comprenant 1 mol/L de MgCl₂, à un débit de 3,5 mL/min ;
** **Étape 4 :*** une colonne 50 de BV égal à 0,63 mL (préalablement lavée avec 4 mL d'une solution aqueuse à 1,0 mol/L de MgCl₂, à un débit de 3,5 mL/min), contenant 235 ± 15 mg de résine PB et que l'on a chargée avec l'éluat E2 obtenu à l'étape 3, à un débit de 3,5 mL/min ;
** **Étape 5 :*** 2 fois 3 mL d'une solution aqueuse A2 comprenant 1,0 mol/L de MgCl₂, à un débit de 3,5 mL/min ;
** **Étape 6 :*** 6 mL d'une solution aqueuse A3 comprenant 0,4 mol/L d'acétate d'ammonium et 75 mmol/L d'acide citrique, à un débit de 3,5 mL/min.

Une solution aqueuse contenant 75,7 MBq de plomb-212 a ainsi été obtenue.

Ce plomb-212 a présenté, vis-à-vis du radium-224, une pureté radiologique de plus de 99,999 %, le radium-224 n'ayant pas été détecté après une semaine.

### Exemple 3 - MgCl₂ 1 M + 1 mM HCl / CLG BV = 1,25 mL / CLL BV = 0,173 mL :

Les étapes 3 à 6 du premier mode de mise en œuvre décrit ci-avant ont été mises en œuvre à la suite de l'Exemple 1 en utilisant :
** **Étape 3 :*** après 24 heures pendant lesquelles on a laissé le radium-224 produire du plomb-212, 10 mL d'une solution aqueuse A1 comprenant 1 mol/L de MgCl₂ et 1 mmol/L de HCl, à un débit de 0,5 mL/min ;
** **Étape 4 :*** une colonne 50 de BV égal à 0,173 mL (préalablement lavée avec 1 mL d'une solution aqueuse à 1,0 mol/L de MgCl₂ et 1 mmol/L de HCl, à un débit de 0,5 mL/min), contenant 73 ± 5 mg de résine PB et que l'on a chargée avec l'éluat E2 obtenu à l'étape 3, à un débit de 0,5 mL/min ;
** **Étape 5 :*** 2 fois 1,5 mL d'une solution aqueuse A2 comprenant 1,0 mol/L de MgCl₂ et 1 mmol/L de HCl, à un débit de 0,5 mL/min ;
** **Étape 6 :*** 1,6 mL d'une solution aqueuse A3 comprenant 0,4 mol/L d'acétate d'ammonium et 75 mmol/L d'acide citrique, à un débit de 0,5 mL/min.

Une solution aqueuse contenant 77,5 MBq de plomb-212 a ainsi été obtenue.

Ce plomb-212 a présenté, vis-à-vis du radium-224, une pureté radiologique de plus de 99,999 %, le radium-224 n'ayant pas été détecté après une semaine.

### Exemple 4 - MgCl₂ 1 M + 1 mM HCl / CLG BV = 5,0 mL / CLL BV = 0,63 mL :

Les étapes 3 à 6 du premier mode de mise en œuvre décrit ci-avant ont été mises en œuvre à la suite de l'Exemple 2 en utilisant :
** **Étape 3 :*** après 71 heures pendant lesquelles on a laissé le radium-224 produire du plomb-212, 30 mL d'une solution aqueuse A1 comprenant 1 mol/L de MgCl₂ et 1 mmol/L de HCl, à un débit de 3,5 mL/min ;
** **Étape 4 :*** une colonne 50 de BV égal à 0,63 mL (préalablement lavée avec 4 mL d'une solution aqueuse à 1,0 mol/L de MgCl₂ et 1 mmol/L de HCl, à un débit de 3,5 mL/min), contenant 235 ± 15 mg de résine PB et que l'on a chargée avec l'éluat E2 obtenu à l'étape 3, à un débit de 3,5 mL/min ;
** **Étape 5 :*** 2 fois 3 mL d'une solution aqueuse A2 comprenant 1,0 mol/L de MgCl₂ et 1 mmol/L de HCl, à un débit de 3,5 mL/min ;
** **Étape 6 :*** 6 mL d'une solution aqueuse A3 comprenant 0,4 mol/L d'acétate d'ammonium et 75 mmol/L d'acide citrique, à un débit de 3,5 mL/min.

Une solution aqueuse contenant 51,5 MBq de plomb-212 a ainsi été obtenue.

Ce plomb-212 a présenté, vis-à-vis du radium-224, une pureté radiologique de plus de 99,999 %, le radium-224 n'ayant pas été détecté après une semaine.

En résumé des exemples 1 à 4, il a été obtenu des rendements de Pb-212 collecté par rapport au plomb produit par décroissance de 88, 77, 95, 85 % respectivement. Les puretés sont supérieures à 99.999 %, le calcul de la pureté prenant en compte les valeurs de limite de détection car le Ra-224 n'a pas été détecté.

### Références citées

**[1]** WO-A-2013/174949
**[2]** WO-A-2017/093069

## Revendications

1. Procédé de production d'une solution aqueuse de plomb-212 de qualité médicale, qui comprend au moins les étapes de :
a) production de plomb-212 par désintégration radioactive du radium-224 présent dans au moins une première colonne de chromatographie (30) contenant une première phase stationnaire (40) sur laquelle est fixé le radium-224 ;
b) élution du plomb-212 ainsi produit de la première phase stationnaire pour obtenir un éluat comprenant du plomb-212 non purifié ;
c) chargement de l'éluat ainsi obtenu dans une deuxième colonne de chromatographie (50) contenant une deuxième phase stationnaire (60) pour fixer le plomb-212 présent dans l'éluat sur la deuxième phase stationnaire ;
d) lavage(s) de la deuxième phase stationnaire pour éliminer les impuretés radioactives susceptibles d'avoir été retenues par la deuxième phase stationnaire sans éliminer le plomb-212 ; puis
e) élution du plomb-212 de la deuxième phase stationnaire, moyennant quoi le plomb-212 de qualité médicale est obtenu en solution aqueuse ;
et qui est **caractérisé en ce que** :
- l'étape b) comprend une circulation dans ladite au moins une première colonne de chromatographie (30) d'une solution aqueuse A1 comprenant de 0,8 mol/L à 1,6 mol/L d'un chlorure, seul ou en mélange avec au plus 200 mmol/L d'acide chlorhydrique ; et
- l'étape d) comprend une circulation dans la deuxième colonne de chromatographie (50) d'une solution aqueuse A2 comprenant de 0,01 mol/L à 1 mol/L du chlorure.

2. Procédé selon la revendication 1, dans lequel le chlorure présent dans les solutions aqueuses A1 et A2 est un chlorure d'un métal alcalin, un chlorure d'un métal alcalino-terreux ou du chlorure d'ammonium.

3. Procédé selon la revendication 2, dans lequel le chlorure est du chlorure de magnésium.

4. Procédé selon l'une quelconque des revendications 1 à 3, dans lequel la solution aqueuse A1 comprend de 0,8 mol/L à 1,2 mol/L et, de préférence, 1,0 mol/L de chlorure de magnésium, seul ou en mélange avec au plus 50 mmol/L d'acide chlorhydrique.

5. Procédé selon l'une quelconque des revendications 1 à 4, dans lequel la solution aqueuse A2 comprend de 0,1 mol/L à 1 mol/L et, de préférence, 1 mol/L de chlorure de magnésium.

6. Procédé selon l'une quelconque des revendications 1 à 5, dans lequel la deuxième colonne de chromatographie (50) présente une tête de colonne et une queue de colonne et l'étape d) comprend la circulation d'un premier volume de solution aqueuse A2 de la tête de colonne vers la queue de colonne, puis la circulation d'un second volume de solution aqueuse A2 de la queue de colonne vers la tête de colonne.

7. Procédé selon l'une quelconque des revendications 1 à 6, dans lequel l'étape e) comprend une circulation dans la deuxième colonne de chromatographie (50) d'une solution aqueuse A3 de pH compris entre 5 et 9 et comprenant un ou plusieurs agents complexants et/ou agents antioxydants.

8. Procédé selon la revendication 7, dans lequel la solution aqueuse A3 comprend de l'acétate d'ammonium et de l'acide citrique et/ou du DOTAM.

9. Procédé selon la revendication 8, dans lequel la solution aqueuse A3 comprend 0,4 mol/L d'acétate d'ammonium et 75 mmol/L d'acide citrique.

10. Procédé selon l'une quelconque des revendications 7 à 9, dans lequel la deuxième colonne de chromatographie (50) présente une tête de colonne et une queue de colonne et la solution aqueuse A3 circule dans la deuxième colonne de chromatographie de la queue de colonne vers la tête de colonne.

11. Procédé selon l'une quelconque des revendications 1 à 10, dans lequel on utilise *m* premières colonnes de chromatographie disposées en parallèle et contenant chacune une première phase stationnaire sur laquelle est fixé le radium-224, *m* étant un nombre entier au moins égal à 2, typiquement compris entre 2 et 4, avantageusement égal à 2 ou 3, et on réalise :
- les étapes a) et b) dans chacune des *m* premières colonnes de chromatographie, moyennant quoi on obtient *m* éluats comprenant du plomb-212 non purifié que l'on collecte séparément ou ensemble pour former un mélange des *m* éluats ;
- l'étape c) en chargeant les *m* éluats ou le mélange des *m* éluats ainsi obtenus dans la deuxième colonne de chromatographie contenant la deuxième phase stationnaire ;
- l'étape d) de lavage de la deuxième phase stationnaire ; et
- l'étape e) d'élution du plomb-212 de la deuxième phase stationnaire.

12. Procédé selon l'une quelconque des revendications 1 à 10, dans lequel on utilise *m* premières colonnes de chromatographie connectées en série et contenant chacune une première phase stationnaire sur laquelle est fixé le radium-224, *m* étant un nombre entier au moins égal à 2, typiquement compris entre 2 et 4, avantageusement égal à 2 ou 3, et on réalise :
- les étapes a) et b) dans chacune des *m* premières colonnes de chromatographie, l'étape b) étant réalisée en faisant circuler la solution aqueuse A1 successivement dans les *m* premières colonnes de chromatographie, moyennant quoi on obtient, au sortir de la *m*^{ième} des *m* premières colonnes de chromatographie, un éluat comprenant du plomb-212 non purifié ;
- l'étape c) en chargeant l'éluat ainsi obtenu dans la deuxième colonne de chromatographie contenant la deuxième phase stationnaire ;
- l'étape d) de lavage de la deuxième phase stationnaire ; et
- l'étape e) d'élution du plomb-212 de la deuxième phase stationnaire.

13. Procédé selon l'une quelconque des revendications 1 à 12, qui comprend de plus, avant l'étape a), les étapes de :
i) production de radium-224 par désintégration radioactive du thorium-228 dans au moins une troisième colonne de chromatographie (10) comprenant une troisième phase stationnaire (20) sur laquelle est fixé le thorium-228 ; et
ii) élution du radium-224 ainsi produit de la troisième phase stationnaire pour obtenir un éluat comprenant du radium-224, l'élution comprenant une circulation dans ladite au moins une troisième colonne de chromatographie d'une solution aqueuse A0 comprenant de 0,4 mol/L à 1 mol/L d'acide nitrique et avantageusement 0,5 mol/L d'acide nitrique ; puis
iii) chargement de l'éluat ainsi obtenu dans ladite au moins une première colonne de chromatographie (30) pour fixer le radium-224 présent dans l'éluat sur la première phase stationnaire (40).

14. Procédé selon la revendication 13, dans lequel on utilise n troisièmes colonnes de chromatographie disposées en parallèle et contenant chacune une troisième phase stationnaire sur laquelle est fixé le thorium-228, *n* étant un nombre entier au moins égal à 2, typiquement compris entre 2 et 5, avantageusement égal à 2 ou 3, et on réalise :
- les étapes i) et ii) dans chacune des *n* troisièmes colonnes de chromatographie, moyennant quoi on obtient *n* éluats comprenant du radium-224 que l'on collecte séparément ou ensemble pour former un mélange des *n* éluats ; et
- l'étape iii) en chargeant les n éluats ou le mélange des *n* éluats ainsi obtenus dans ladite au moins une première colonne de chromatographie.

15. Procédé selon la revendication 13, dans lequel on utilise *n* troisièmes colonnes de chromatographie connectées en série et contenant chacune une troisième phase stationnaire sur laquelle est fixé le thorium-228, *n* étant un nombre entier au moins égal à 2, typiquement compris entre 2 et 5, avantageusement égal à 2 ou 3, et on réalise :
- les étapes i) et ii) dans chacune des *n* troisièmes colonnes de chromatographie, l'étape ii) étant réalisée en faisant circuler la solution aqueuse A0 successivement dans les *n* troisièmes colonnes de chromatographie, moyennant quoi on obtient, au sortir de la *n*^{ième} des *n* troisièmes colonnes de chromatographie, un éluat comprenant du radium-224 ; et
- l'étape iii) en chargeant l'éluat ainsi obtenu dans ladite au moins une première colonne de chromatographie.
